# EUROPÄISCHE PATENTSCHRIFT

(11) **EP 0 659 079 B1**
(45) Veröffentlichungstag und Bekanntmachung des Hinweises auf die Patenterteilung: **22.01.1997**
(21) Anmeldenummer: 93919071.6
(22) Anmeldetag: 16.08.1993
(51) Int. Cl.: A61K 31/55, A61K 9/70, A61K 9/00

(54) **TRANSDERMALES THERAPEUTISCHES SYSTEM MIT PENTYLENTETRAZOL ALS WIRKSTOFF**
TRANSDERMAL THERAPEUTIC SYSTEM WITH PENTYLENE TETRAZOL AS ACTIVE SUBSTANCE
SYSTEME THERAPEUTIQUE PERCUTANE CONTENANT DU PENTYLENETETRAZOL COMME PRINCIPE ACTIF

(30) Priorität: 02.09.1992 DE 4229230
(43) Veröffentlichungstag der Anmeldung: 28.06.1995
(73) Patentinhaber: LTS LOHMANN Therapie-Systeme GmbH, 56567 Neuwied (DE)
(72) Erfinder: HERRMANN, Fritz, D-56567 Neuwied (DE); HILLE, Thomas, D-56564 Neuwied (DE)
(74) Vertreter: Flaccus, Rolf-Dieter, Dr.
(86) Internationale Anmeldenummer: EP9302183
(87) Internationale Veröffentlichungsnummer: WO9405295

(56) Entgegenhaltungen:
- NEUROPHARMACOLOGY, Bd. 14, Nr. 11, 1975, Seiten 869-881; R.M.JOY: 'Comparative effects of convulsants on the antidromic cortical response to pyramidal tract stimulation'
- J. PHYSIOL., Bd. 372, 1986, Seiten 445-456; W.FELDBERG ET AL.: 'Blood pressure effects of leptazol applied to the ventral surface of the brain stems of cats'
- J.PHYSIOL., Bd. 347, 1984, Seiten 345-360; P.G.GUERTZENSTEIN ET AL.: 'Cardiovascular responses evoked from the nicotine-sensitive area on the ventral surface of the medulla oblongata in the cat'
- PHARMACOL. BIOCHEM. BEHAV., Bd. 32, Nr. 3, 1989, Seiten 667-670; MICHAEL J. DURCAN ET AL.: 'Reduction of the intoxicating effects of ethanol by drugs acting at the benzodiazepine-GABA receptor complex', see abstract
- AM. J. PHYSIOL., Bd. 234, Nr. 5, 1978, Seiten H582-H591; HERMES A. KONTOS ET AL.: 'Role of tissue hypoxia in local regulation of cerebral microcirculation', see abstract

## Beschreibung

Die Erfindung betrifft ein pharmazeutisches Produkt mit Anteilen von Pentetrazol als Wirkstoff für eine transdermale Applikation zur Prophylaxe und Behandlung von Vasodilatationen oder Befindlichkeitsstörungen , die durch anfallartige Erweiterung der Blutgefäße im Gehirn verursacht werden. Solche Störungen sind beispielsweise Migräne und Befindlichkeitsstörungen, die durch übermäßigen Alkoholkonsum ("Kater") hervorgerufen werden.

Beide, Migräne und "Kater", hoben gemeinsam, daß die Beschwerden ihre Ursache in Blutgefäßerweiterungen im Gehirn hoben. Während die Gefäßerweiterung, die zum "Kater" führt, auf die dilatierende Wirkung des Ethanols zurückzuführen ist, ist im Falle der Migräne die Ursache noch unbekannt.
Trotz der mit Migräne verbundenen menschlichen Probleme und des immensen volkswirtschaftlichen Schadens stagniert die Innovation der medikamentösen Behandlung dieser Krankheit. Spezielle Mittel zur Behandlung des "Katers" werden in der Patentliteratur zwar immer wieder beschrieben (z.B. EP 0 271 489), meist handelt es sich hierbei aber nur um relativ willkürliche Kombinationen aus schwachen Analgetika, Vitaminen, Provitaminen, Fructose und Antihistaminica.
Schwach wirksame Analgetika, im allgemeinen auch "Kopfschmerzmittel" genannt, beseitigen in beiden Fällen nicht die Ursache des Unwahlbefindens, nämlich die Gefäßerweiterung, sondern unterdrücken das Symptom, den Kopfschmerz. Im Falle von Migräne besteht die Gefahr zur überhöhten Dosierung, was für das Eintreten von Langzeitschäden durch diese Arzneimittelgruppe verantwortlich ist.

Gegen Übelkeit und Erbrechen werden Metoclopramid, Bromoprid und Domperidon eingesetzt. Auch für diese Symptome gilt das gleiche, was oben für das Symptom "Kopfschmerz" ausgeführt wurde.

Es ist bekannt, schwer und langandauernde Migräneanfälle mit genuinen und hydrierten Mutterkornalkaloiden zu therapieren, vor allem mit Ergotamin oder Dihydroergotamin. Wird die Behandlung der Migräne im Intervall vorgenommen, lassen sich Zahl und Schwere der Migräneanfälle verringern.

Daneben bestehen auch nichtmedikamentöse Behandlungsmöglichkeiten. Zu diesen Maßnahmen zählen das Vermeiden übermäßiger Nahrungs- und Flüssigkeitsaufnahme am Abend, geregelter Schlaf und eine Einschränkung des Alkoholkonsums. Letztere Variante der Prophylaxe zeigt den Zusammenhang von Migräne und Alkohol deutlich auf.

Bei anderen Formen der Migräne kommen heute weiterhin überwiegend Wirkstoffe aus folgenden Stoffgruppen zum Einsatz:
Betablocker (Propanolol und Metoprolol),
Calciumantagonisten (z.B. Flunarizin),
Serotoninantagonisten.
Thrombozytenaggregationshemmer,
α-Sympatoliytica (z.B. Clonidin) und
Antidepressiva (z.B. Amitriptylin)
(Ditzel, P., Deutsche Apothekerzeitung 14, 690 (1992).

Schon die Verschiedenheit der aufgeführten Arzneistoffgruppen zeigt, daß es kein Mittel zur prophylaktischen Behandlung aller Formen der Migräne gibt. Es werden auch keine der aufgeführten Pharmaka zur Prophylaxe und Behandlung der Folgen übermäßigen Alkoholkonsums eingesetzt.

Die Prophylaxe ist aber das wesentliche, wenn Migräne oder "Kater" vermieden werden sollen. Dies ist aber das entscheidende, denn es ist bekannt, daß die rechtzeitige Gabe von Migränemitteln einen Anfall verhindert. Migränemittel sind aber mit so vielen Nebenwirkungen behaftet, daß sich bisher ein Ennehmen "auf Verdacht" verbot.

Aus dem Dokument J.Physiol.: Band 372, 1986, Seite 445 bis 456, insbesondere Zusammenfassung der Seite 445 sowie Seite 447, 2. Absatz und
aus dem Dokument Neuropharmacoly, Band 14, Nr. 11, 1975, Seiten 869 bis 881, insbesondere Zusammenfassung und Seite 871, 3. Absatz sind therapeutische Zusammensetzungen enthaltend Pentetrazol und einen hautverträglichen Hilfsstoff sowie deren topische Anwendung bekannt. Beschrieben werden darin Tierversuche mit ausschließlicher Applikation durch paarweise Perspex-Ringe oder durch Injektionen sowie durch Kanülen in brachiocevale Venen mittels Infusionen.

Aufgabe der Erfindung ist daher die Bereitstellung eines pharmazeutischen Produktes für eine transdermale Applikation zur Prophylaxe und Behandlung von Vasodilatation, die sich in Migräneanfällen und "Kateranfällen" äußert. Dabei soll die Applikationsweise nicht die genannten Nachteile des Standes der Technik aufweisen und insbesondere geringe, aber konstante Plasmaspiegel liefern.

Diese Aufgabe wird gelöst durch ein pharmazeutisches Produkt nach Anspruch 1.

Ein Herstellungsverfahren ist entsprechend dem Anspruch 9 vorgesehen. Weitere Ausgestaltungen des Produktes bzw. der Herstellung sind entsprechend den Unteransprüchen vorgesehen.

Pentetrazol ist ein zentrales Analepticum und gehört nach seinen Angriffspunkten zu den sog. Stammhirnanaleptika. Wirkort ist dabei die Substancia reticularis, in der auch die medullären Zentren für Atmung und Kreislauf liegen. Psychostimulierende Wirkungen sind dabei nicht vorhanden. Pentetrazol hat keine direkten peripheren Wirkungen. Bei schwerer Barbituratvergiftung mit Gefähr der Atemlähmung kann die Gabe der Substanz als erste Hilfe nützlich sein.

Die Wirkung des Pentetrazols kommt allein durch die Erregung zentraler, vegetativer Regionen zustande, insbesondere des Vasomotorenzentrums (Europäisches Arzneibuch Band III Kommentar, 2. verbesserte Auflage 1982 Wissenschaftliche Verlagsgesellschaft mbH Stuttgart Govi-Verlag GmbH Frankfurt).

Der Wirkstoff Pentetrazol stellt ein System aus zwei kondensierenden Ringen dar. Beteiligt sind der durchhydrierte Azepinring und Tetrazol. Pentetrazol ist farblos und kristallin, leicht löslich in Wasser, Ethanol, Chloroform und chemisch sehr stabil. Aufgrund des niedrigen Schmelzpunktes von 58°C bis 60°C findet leicht Verklumpung statt. Da im Pentetrazol keine NH-Funktion vorhanden ist, besitzt es auch keine sauren Eigenschaften mehr. Die

Basizität ist so gering, daß eine Protonierung nur mit starken Säuren in wasserfreiem Medium gelingt.

Die hier beschriebene Verwendbarkeit von Pentetrazol ist überraschend, weil die pharmakologischen Wirkungen des Pentetrazols zwar intensiv untersucht worden sind, ein prophylaktischer Effekt von Pentetrazol in der Migränetherapie aber bisher nicht beschrieben wurde.

In der Therapie von Alkoholvergiftungen wurde der Wirkstoff bisher nur zur Behandlung der Atemlähmung, der Todesursache bei Ethanolintoxikationen, eingesetzt. Bei diesem Verwendungszweck wird jedoch die geringe therapeutische Breite von Pentetrazol moniert (Erickson, C.K. Adv. Exp. med. Biol. 126, 5512 (1980).

Aufgrund dieser geringen therapeutischen Breite muß auch für Pentetrazol eine Applikationsform gefunden werden, die zwar geringe aber konstante Plasmaspiegel liefert, denn Medikamente zur Prophylaxe müssen rechtzeitig, d.h. auch bei Verdacht, gegeben werden. Übliche Gaben über die orale Route führen nicht zu niedrigen, konstanten Blutspiegeln. Besser geeignet ist die transdermale Route, wobei die Arzneiform eine Salbe, Creme, Lotion oder ein transdermales therapeutisches System (TTS) sein kann.

Unter Salben und Cremen werden dabei halbfeste Arzneiformen zum Einreiben in die Haut verstanden, wobei "Salbe" der Oberbegriff ist, da definitionsgemäß Salbe aus Salbengrundlage und Arzneistoff bestehen, während Creme W/O- oder O/W-Emulsionen darstellen. Bei Lotionen handelt es sich dagegen um Schüttelmixturen (=Suspensionen) zum Einreiben in die Haut auf meist wässriger Basis. Hilfsstoffe zur Herstellung von Salbe, Cremen und Lotionen sind dem Fachmann bekannt. Eine Steuerung der Wirkstoffabgabe findet nicht statt, vielmehr ist die Aufnahmefähigkeit der Haut für den Wirkstoff der limitierende Faktor. Anders dagegen beim TTS. Dieses ist eine auf die Haut aufzubringende Applikationsform mit dem Aussehen traditioneller Pflaster, die über die Haut abzugebende Wirkstoffe enthalten. Ein therapeutisches System kann einen oder mehrere Wirkstoffe enthalten, die in vorausbestimmter Rate kontinuierlich über einen festgesetzten Zeitraum an einen festgelegten Anwendungsort abgegeben werden ("Heilmann, Klaus:

Therapeutische Systeme - Konzept und Realisation programmierter Arzneiverabreichung", 4. Auflage, Ferdinand Enke Verlag Stuttgart, 1984, S. 26.).

TTS gemäß vorliegender Erfindung sind Pflaster mit wirkstoffundurchlässiger Rückschicht, einem damit verbundenen Wirkstoffreservoir aus einer Polymermatrix, bei Abwesenheit anderer Steuermechanismen einer die Abgabe des Wirkstoffs steuernde Membran, einer Haftklebereinrichtung zur Befestigung des Pflasters auf der Haut und im Bedarfsfall mit einer vor der Applikation des Pflasters wieder ablösbaren Schutzschicht.

Geeignete Pflaster sind beispielsweise in dem Deutschen Patent DE 33 15 272 beschrieben. Im Prinzip sind auch alle Pflasterformen geeignet, wie beispielsweise in der DE-A-41 10 027.1-35 beschrieben.
Die in dieser Erfindung bevorzugte Form des TTS ist die Variante Matrixsystem, bestehend aus wirkstoffundurchlässiger Rückschicht, wirkstoffenthaltender selbstklebender Reservoirschicht und einer wiederablösbaren Schutzschicht.

Die wirkstoffundurchlässige Rückschicht kann aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden. Es können auch textile Flächengebilde verwendet werden, wenn die Bestandteile des Reservoirs aufgrund ihrer physikalischen Beschaffenheit durch sie nicht hindurchtreten können. Bei einer bevorzugten Ausführungsform ist die Rückschicht ein Verbundstoff aus einer mit Aluminium bedampften Folie.

Die Reservoirschicht enthält eine Polymermatrix und den Wirkstoff, wobei die Polymermatrix den Zusammenhalt des Systems gewährleistet. Sie besteht aus einem Grundpolymer und ggf. den üblichen Zusätzen.

Die Auswahl des Grundpolymers richtet sich nach den chemischen und physikalischen Eigenschaften des Pentetrazols. Beispielhafte Polymere sind Kautschuk, kautschukähnliche, synthetische Homo-, Co- oder Blockpolymere, Poly(meth)acrylsäuren und Poly(meth)acrylsäureester und deren Copolymere, Polyurethane und Silikone. Grundsätzlich kommen alle Polymere in Frage, die zur Herstellung von Haftklebern eingesetzt werden können und physiologisch unbedenklich sind. Besonders bevorzugt sind solche, die aus Blockcopolymeren auf Basis von Styrol und 1,3-Dienen, Polyisobutylenen. Polymeren auf Acrylat- und/oder Methacrylat bestehen.

Von den Blockcopolymeren auf Basis von Styrol und 1,3-Dienen werden ganz besonders lineare Styrol-Isopren- oder Styrol-Butadien-Blockcopolymere eingesetzt.

Als Polymere auf Acrylatbasis werden selbstvernetzende Acrylatcopolymere aus 2-Ethylhexylacrylat, Vinylacetat und Acrylsäure mit, bzw. nicht selbstvernetzende Acrylatcopolymere ohne, Titanchelatester bevorzugt.

Als Polymere, die dem Grundpolymer zugesetzt werden können, kommen Polymethacrylate, Ester von hydriertem Kolophonium und Polyvinyle in Frage.

Als Methacrylate werden Copolymere auf Basis von Dimethylaminoethylmethacrylaten und neutralen Methacrylsäureestern bevorzugt. Als Ester von hydriertem Kolophonium werden vorzugsweise insbesondere dessen Methyl- und Glycerinester verwendet. Als Polyolefine werden vorzugsweise Polyisobutylene und Polyvinylalkohole eingesetzt.

Die Art der üblichen Zusätze hängt vom eingesetzten Polymer ab: Nach ihrer Funktion lassen sie sich einteilen in beispielsweise Klebrigmacher, Stabilisatoren, Trägerstoffe, Weichmacher und Füllstoffe. Die hierfür in Frage kommenden physiologisch unbedenklichen Substanzen sind dem Fachmann bekannt.

Die Wahl des Weichmachers richtet sich nach dem Polymer. Besonders geeignet sind höhere Alkohole wie Dodecanol, 2-Octyldodecanol, Undecanol, Octanol, Ester von Carbonsäure, wobei die Alkoholkomponente auch ein polyethoxylierter Alkohol sein kann, Diester von Dicarbonsäure, z.B. Di-n-butyladipat sowie Triglyceride, insbesondere mittelkettige Triglyceride der Capryl/Caprinsäuren des Kokosöls.

Weitere Beispiele für einen geeigneten Weichmacher sind mehrwertige Alkohole, z.B. Glycerin und Propandiol-(1,2) u.a., die auch durch Polyethylenglykole verestert sein können. Weitere Beispiele sind gesättigte oder ungesättigte, cyclische oder lineare Kohlenwasserstoffe wie z.B. 2,6 Dioctylcyclohexan.

Die Reservoirschicht besitzt eine solche Eigenklebrigkeit, daß ein dauernder Kontakt zur Haut sichergestellt ist. Die Konzentration an Pentetrazol hängt von der Größe der Fläche des Reservoirs ab. Sie kann zwischen 0.1 bis 50 Gew.-% der Polymermatrix betragen.

Die ablösbare Schutzschicht, die mit der Reservoirschicht in Berührung steht und vor der Anwendung entfernt wird, besteht bespielsweise aus denselben Materialien, wie sie zur Herstellung der Rückschicht benutzt werden, vorausgesetzt, daß sie ablösbar gemacht werden, wie z.B. durch eine Siliconbehandlung. Andere ablösbare Schutzschichten sind z.B. Polytetrafluorethylen, behandeltes Papier, Cellophan, Polyvinylchlorid u.ä. Wird das erfindungsgemäße Laminat nach Aufbringen der Schutzschicht in therapiegerechte Formate (Pflaster) aufgeteilt, so können die dann aufzubringenden Schutzsschichtformate ein überstehendes Ende aufweisen, mit dessen Hilfe sie leichter von dem Pflaster abgezogen werden können.

Das erfindungsgemäße transdermale therapeutische System wird hergestellt, indem Wirkstoff, Weichmacher und fallweise Hilfsstoffe zusammen mit Bestandteilen der haftklebenden Reservoirschicht in Lösung homogen vermischt und auf die wirkstoffundurchlässige Rückschicht aufgestrichen wird, worauf Lösemittel entfernt wird. Anschließend wird die Klebeschicht mit einer Schutzschicht versehen.

Auch der umgekehrte Weg, daß die Kleberlösung auf die Schutzschicht aufgestrichen wird, ist grundsätzlich möglich. Man entfernt auch in diesem Fall die Lösemittel und deckt dann anschließend mit der Rückschicht ab.
Grundsätzlich läßt sich das Reservoir auch aus der Schmelze herstellen.

Die Herstellung des Produktes wird wie folgt erläutert:

Pentetrazol, Weichmacher und ggf. andere Hilfsstoffe werden unter Rühren bei Raumtemperatur in einer Haftkleberlösung gelöst. Nach vollständiger Auflösung streicht man die erhaltene Lösung auf eine durch Silikonbeschichtung wieder ablösbar gemachte Polyesterfolie mit der Dicke 100 µm. Durch Verwendung eines geeigneten Rakels wird ein Flächengewicht von 125 g/m² eingestellt. Nachdem die Lösemittel durch 10 minütiges Trocknen im Trockenschrank bei 80° entfernt wurden, deckt man den Haftkleberfilm mit einer 15 µm dicken Polyesterfolie ob und stanzt mit einem geeigneten Schneidewerkzeug 16cm² große Pflaster aus. Noch Abziehen der silikonisierten Polyesterfolie klebt man die Pflaster auf Mäusehäute und bestimmt die Penetrationsrate in einer Diffusionszelle noch Chien (Kestrary, P.R., Huag, Y.C., Chien, Y.W., Drug Develp. & Ind. Pharm. 11, 1213-1254 (1985). Als Akzeptormedium dient 0,9 %-ige Kochsalzlösung. Nach 8h bzw. 24 h wird die Konzentration des Pentetrazols im Akzeptormedium per HPLC bestimmt. In der folgenden Tabelle sind die Rezepturbestandteile und die Penetrationsraten der Pentetrazol-TTS angegeben.

Im folgenden werden Rezepturbestandteile und Penetrationsvermögen in den Beispielen 1 bis 10 aufgezeigt:
1. Als Basisstoffe für die Matrix werden verwendet:
   - saures Polyacrylat:: Eingesetzt wurde selbstvernetzendes Acrylatcopolymerisat aus 2-Ethylhexylacrylat, Vinylacetat und Acrylsäure.
   Die Säurezahl beträgt 40.
   - basisches Methacrylat:: Eingesetzt wurde ein Copolymerisat mit kationischem Charakter auf Basis von Dimethylaminoethylmethacrylat und neutralen Methacrylsäureestern. Das mittlere Molekulargewicht liegt bei 150000.
   Die KOH-Zahl beträgt ca. 180 (162-198)
   - neutrales Methacrylat:: Eingesetzt wurde ein Copolymerisat mit neutralem, nichtionischem Charakter auf Basis von Methacrylsäuremethylester und Methacrylsäurebutylester. Das mittlere Molekulargewicht liegt bei 200000. Die Säurezahl beträgt max. 1,0.
2. Die folgenden Beispiele belegen die gute Hautgängigkeit des Pentetrazols, da fast die gesamte verfügbare Menge an Pentetrazol durch die Haut diffundiert. Damit ist die wichtigste Voraussetzung für eine gesteuerte transdermale Applikation gegeben.

| Bsp. Nr. | Pentetrazol | Weichmacher | Haftkleber | Hilfsstoff | Flux an der Mäusehaut | |
|---|---|---|---|---|---|---|
| | | | | | mg PTZ/cm² x 8h | mg PTZ/cm² x 24h |
| 1 | 20 mg | 15 mg 2,6-Dioctylcyclohexan | 165 mg saures Polyacrylat | ---- | 0,73 | 1,2 |
| 2 | 20 mg | 15 mg 2-Octyldodecanol | 165 mg saures Polyacrylat | ---- | 0,71 | 1,19 |
| 3 | 20 mg | 15 mg 1-Dodecanol | 165 mg saures Polyacrylat | ---- | 0,74 | 1,25 |
| 4 | 20 mg | 15 mg n-Dibutyladipat | 165 mg saures Polyacrylat | ---- | 0,66 | 1,25 |
| 5 | 20 mg | 15 mg Triglyceride mittelkettiger Fettsäuren | 165 mg saures Polyacrylat | ---- | 0,53 | 0,88 |
| 6 | 20 mg | 15 mg polyethoxyliertes Glycerin mit C8/C10 Ethoxygruppen, deren freie Hydroxylgruppen teilweise mit Capryl/Caprinsäuren verestert sind | 165 mg saures Polyacrylat | ---- | 0,57 | 1,06 |
| 7 | 20 mg | 10 mg 2,6-Dioctylcyclohexan | 165 mg saures Polyacrylat | ---- | 0,54 | 0,88 |
| 8 | 20 mg | 10 mg 1-Dodecanol | 165 mg saures Polyacrylat | ---- | 0,76 | 1,25 |
| 9 | 20 mg | 15 mg Triglyceride mittelkettiger Fettsäuren | 165 mg saures Polyacrylat | ---- | 0,55 | 0,95 |
| 10 | 20 mg | 10 mg polyethoxyliertes Glycerin mit C8/C10 ethoxygruppen, deren freie Hydroxylgruppen teilweise mit Capryl/Caprinsäuren verestert sind | 165 mg saures Polyacrylat | ---- | 0,65 | 1,18 |

## Patentansprüche

1. Pharmazeutisches Produkt mit Anteilen von Pentetrazol (6, 7, 8, 9-Tetrahydro-5H-tetrazol (1,5a) azepin), als Wirkstoff, dadurch gekennzeichnet,daß es in einer für eine transdermale Applikation geeigneten Form als Pflaster mit einer undurchlässigen Rückschicht, einem damit verbundenen Wirkstoffreservoir aus einer Polymermatrix, bei Abwesenheit anderer Steuermechanismen einer die Abgabe des Wirkstoffs steuernden Membran, einer Haftklebeeinrichtung zur Befestigung des Pflasters auf der Haut und im Bedarfsfall mit einer vor Applikation des Pflasters ablösbaren Schutzschicht ausgebildet ist.

2. Pharmazeutisches Produkt nach Anspruch 1, dadurch gekennzeichnet, daß es mindestens einen hautverträglichen Hilfsstoff enthält.

3. Pharmazeutisches Produkt nach Anspruch 1 oder 2 zur Verwendung zur Prophylaxe von pathologischen Vasodilatationen.

4. Pharmazeutisches Produkt nach Anspruch 1 oder 2 zur Verwendung zur Behandlung von pathologischen Vasodilatationen.

5. Pharmazeutisches Produkt nach einem oder mehreren der Ansprüche 1 bis 4, dadurch gekennzeichnet, daß es 0,1 bis 50 Gew.-%, vorzugsweise 3 bis 25 Gew.-% und besonders bevorzugt 5 bis 15 Gew.-% Wirkstoff enthält.

6. Verfahren zur Herstellung des pharmazeutischen Produktes für eine transdermale Applikation zur Prophylaxe und Behandlung von Vasodilatationen nach den Ansprüchen 1 bis 5 , gekennzeichnet durch die Arbeitsschritte:
a) Wirkstoff, Weichmacher und fallweise Hilfsstoffe werden zusammen mit Bestandteilen einer Haftkleberlösung miteinander in Lösung homogen vermischt,
b) die Lösung wird auf eine wirkstoffundurchlässige Rückschicht in gleichmäßig dünner Schicht aufgetragen,
c) sodann wird durch Wärmezufuhr das Lösemittel ausgetrieben und
d) die gebildete haftklebende Schicht mit einer dehäsiv ausgerüsteten Schutzschicht abgedeckt, woraufhin aus dem Laminat
e) Einzelpflaster durch Ausstanzen konfektioniert werden.

7. Verfahren noch Anspruch 6, dadurch gekennzeichnet, daß die Konzentration des Wirkstoffes Pentetrazol zwischen 0,1 und 50 Gew.-% der Polymermatrix beträgt.

8. Verfahren nach den Ansprüchen 6 und 7, dadurch gekennzeichnet, daß als Grundpolymer für die Matrix Kautschuk, kautschukähnliche. synthetische Homo-, Co- oder Blockpolymere, Poly(meth)acrylsäuren und Poly(meth)acrylsäureester und deren Copolymere, Polyurethane und Silikone verwendet werden.

9. Verfahren nach Anspruch 7, dadurch gekennzeichnet, daß als Grundpolymer bevorzugt Blockcopolymere auf Basis von Styrol und 1,3-Dienen, Polyisobutylenen, Polymeren auf Acrylat- und/oder Methacrylat verwendet werden.

10. Verfahren nach einem der Ansprüche 6 bis 9, dadurch gekennzeichnet, daß als Polymere, die dem Grundpolymer zugesetzt werden, Polymethacrylate, Ester von hydriertem Kolophonium und Polyvinyle verwendet werden.

11. Verfahren nach einem oder mehreren der Ansprüche 6 bis 10, dadurch gekennzeichnet, daß als Weichmacher höhere Alkohole wie Dodecanol, 2-Octyldodecanol, Undecanol, Octanol, Ester von Carbonsäure - wobei die Alkoholkomponente auch ein polyethoxylierter Alkohol sein kann - Diester von Dicarbonsäure, z.B. Di-n-butyl-adipat sowie Triglyceride, insbesondere mittelkettige Triglyceride der Capryl/Caprinsäuren des Kokosöls verwendet werden.

12. Verfahren noch Anspruch 11, dadurch gekennzeichnet, daß als Weichmacher bevorzugt mehrwertige Alkohole wie Glycerin und Propandiol-(1,2) u.a., die auch durch Polyethylenglykole verestert sein können, sowie gesättigte oder ungesättigte, cyclische oder lineare Kohlenwasserstoffe wie Dioctylcyclohexan verwendet werden.

13. Verfahren nach einem oder mehreren der Ansprüche 6 bis 12, dadurch gekennzeichnet, daß das Wirkstoffreservoir aus einem der Auftrag de Lösung mit einem Flächengewicht von ca. 125 g/m² gebildet wird.

## Claims

1. Pharmaceutic product comprising portions of pentetrazol (6,7,8,9-tetrahydro-5H-tetrazol(1,5a)azepine) as active substance, characterized in that said pharmaceutic product is present in a form suitable for transdermal application, i.e. as a plaster comprising an impermeable backing layer, an active-substance reservoir of a polymer matrix connected thereto, and, where no other control mechanisms are present, a membrane controlling the release of active substance, a pressure-sensitive adhesive device for securing the plaster to the skin and, if required, a protective layer which is removable prior to application of the plaster.

2. The pharmaceutic product according to claim 1, characterized in that it contains at least one skin-compatible auxiliary agent.

3. The pharmaceutic product according to claim 1 or 2 for use in the prophylaxis of pathologic vasodilations.

4. The pharmaceutic product according to claim 1 or 2 for use in the treatment of pathologic vasodilations.

5. Pharmaceutic product according to one or more of claims 1 to 4, characterized in that it contains 0.1 to 50%-wt., preferably 3 to 25%-wt. and particularly preferred 5 to 15%-wt. of active substance.

6. Process for the production of the pharmaceutic product for a transdermal application for the prophylaxis and treatment of vasodilations according to claims 1 to 5, characterized by the following steps:
a) active substance, plasticizer(s) or softener(s), and, optionally, auxiliary agents are homogeneously mixed, in solution, with the components of a solution of pressure-sensitive adhesive,
b) the solution is applied to an active substance-insoluble backing layer to form an evenly thin layer,
c) then the solvent is driven off by application of heat,
d) the resultant pressure-sensitive adhesive layer is covered with a protective layer which has been rendered dehesive, whereupon
e) individual plasters are manufactured by punching said plasters out of the laminte.

7. The process according to claim 6, characterized in that the concentration of the active substance pentetrazole is between 0.1 and 50%-wt of the polymer matrix.

8. The process according to claims 6 and 7, characterized in that as base polymer of the matrix the following substances are used: rubber; rubber-similar synthetic homopolymers, copolymers or blockpolymers; poly(meth)acrylic acids and poly(meth)acrylic acid esters and the copolymers thereof; polyurethanes and silicones.

9. The process according to claim 7, characterized in that as base polymer the following substances are preferably used: blockcopolymers based on styrene and 1,3-dienes; polyisobutylenes; polymers based on acrylate and/or methacrylate.

10. The process according to any one of claims 6 to 9, characterized in that as polymers which are added to the base polymer polymethacrylates, esters of hydrogenated colophony and polyvinyls are used.

11. The process according to one or more of claims 6 to 10, characterized in that as plasticizers or softeners higher alcohols such as dodecanol, 2-octyldodecanol, undecanol, octanol; esters of carboxylic acid - the alcohol component thereof may also be a polyethoxylated alcohol; diesters of dicarboxylic acid, e.g. di-n-butyl-adipate; as well as triglycerides, in particular medium-chain triglycerides of caprylic/capric acids of coconut oil.

12. The process according to claim 11, characterized in that as plasticizers or softeners the following substances are preferably used: polyvalent alcohols such as glycerin and propanediol-(1,2) etc., which may also be esterified by polyethylene glycols; as well as saturated or unsaturated, cyclic or linear hydrocarbons such as dioctylcyclohexane.

13. The process according to one or more of claims 6 to 12, characterized in that the active substance reservoir is formed by spreading the solution such that a weight per unit area of 125 g/m² is obtained.

## Revendications

1. Produit pharmaceutique avec des proportions de pentétrazole (6,7,8,9-tétrahydro-5H-tétrazole(1,5a)azépine, à titre de principe actif, caractérisé en ce qu'il se présente sous une forme convenant à l'application transdermique, constitué d'un timbre avec une couche dorsale imperméable, un réservoir de principe actif qui y est assujetti et qui est constitué d'une matrice polymérique, avec, en l'absence d'autres mécanismes régulateurs, une membrane régulant la libération du principe actif, un dispositif autocollant pour la fixation du timbre sur la peau et, en cas de besoin, avec une couche protectrice amovible avant l'application du timbre.

2. Produit pharmaceutique suivant la revendication 1, caractérisé en ce qu'il contient au moins un adjuvant compatible avec la peau.

3. Produit pharmaceutique suivant la revendication 1 ou 2, à utiliser pour la prophylaxie de vasodilatations pathologiques.

4. Produit pharmaceutique suivant la revendication 1 ou 2, à utiliser pour le traitement de vasodilatations pathologiques.

5. Produit pharmaceutique suivant une ou plusieurs des revendications 1 à 4, caractérisé en ce qu'il contient de 0,1 à 50% en poids, de préférence, de 3 à 25% en poids et, de manière plus particulière, de 5 à 15% en poids, de principe actif.

6. Procédé de préparation d'un produit pharmaceutique destiné à une application transdermique pour la prophylaxie et le traitement de vasodilatations suivant les revendications 1 à 5, caractérisé en ce qu'il comprend les étapes opératoires suivantes :
a) on mélange de façon homogène le principe actif, le plastifiant et, le cas échéant, les adjuvants ensemble avec les composants d'une solution de colle de contact, en solution,
b) on applique la solution en une mince couche uniforme sur une couche dorsale imperméable au principe actif,
c) on chasse ensuite le solvant par application de chaleur et
d) on recouvre la couche autocollante formée d'une couche protectrice rendue anti-adhésive, puis, à partir du lamifié obtenu,
e) on confectionne des timbres individuels par estampage.

7. Procédé suivant la revendication 6, caractérisé en ce que la concentration du principe actif, à savoir le pentétrazole, constitue de 0,1 à 50% en poids de la matrice polymérique.

8. Procédé suivant les revendications 6 et 7, caractérisé en ce que l'on utilise, à titre de polymère de base pour la matrice, du caoutchouc, des homopolymères, copolymères, ou polymères séquencés, analogues au caoutchouc, synthétiques, des poly(acides (méth)acryliques) et des poly(méth)acrylates et leurs copolymères, des polyuréthannes et des silicones.

9. Procédé suivant la revendication 7, caractérisé en ce que l'on utilise, à titre de polymère de base, de préférence, des copolymères séquencés à base de styrène et de 1,3-diènes, des polyisobutylènes, des polymères d'acrylate et/ou de méthacrylate.

10. Procédé suivant l'une quelconque des revendications 6 à 9, caractérisé en ce que l'on utilise, à titre de polymères que l'on ajoute au polymère de base, des polyméthacrylates, des esters de la colophane hydrogénée et des composés polyvinyliques.

11. Procédé suivant une ou plusieurs des revendications 6 à 10, caractérisé en ce que l'on utilise, à titre de plastifiants, des alcools supérieurs, comme le dodécanol, le 2-octyldodécanol, l'undécanol, l'octanol, des esters d'acides carboxyliques - où le composant alcool peut aussi être un alcool polyéthoxylé - des diesters d'acides dicarboxyliques, par exemple l'adipate de di-n-butyle, ainsi que des triglycérides, plus particulièrement, des triglycérides à chaîne moyenne des acides caprylique/caproïque de l'huile de coco.

12. Procédé suivant la revendication 11, caractérisé en ce que l'on utilise, à titre de plastifiants, de préférence, des alcools polyhydroxylés, comme la glycérine et le propanediol-(1,2) et analogues, qui peuvent aussi être estérifiés par des polyéthylèneglycols, ainsi que des hydrocarbures cycliques ou linéaires, saturés ou insaturés, comme le dioctylcyclohexane.

13. Procédé suivant une ou plusieurs des revendications 6 à 12, caractérisé en ce que le réservoir de principe actif est formé par application de la solution avec un grammage d'environ 125 g/m².
